# FASCICULE DE BREVET EUROPEEN

(11) **EP 0 367 666 B1**
(45) Date de publication et mention de la délivrance du brevet: **20.07.1994**
(21) Numéro de dépôt: 89402984.2
(22) Date de dépôt: 27.10.1989
(51) Int. Cl.: C12Q 1/68, C12N 15/12

(54) **Fragments d'acide nucléique spécifiques du gène de la villine humaine-leur utilisation aux fins de diagnostic**
Spezifische Nukleinsäurefragmente des menschlichen Villingens, ihre Anwendung für diagnostische Zwecke
Specific nucleic-acid fragments of the human villin gene and their application to diagnostic ends

(30) Priorité: 28.10.1988 FR 8814208
(43) Date de publication de la demande: 09.05.1990
(73) Titulaire: INSTITUT PASTEUR, 75724 Paris Cédex 15 (FR)
(72) Inventeur: Arpin, Monique, F-75014 Paris (FR); Pringault, Eric, F-75015 Paris (FR); Garcia, Alphonse, F-75015 Paris (FR); Louvard, Daniel, F-92330 Sceaux (FR)
(74) Mandataire: Gutmann, Ernest

(56) Documents cités:
- EP-A- 0 206 849
- US-A- 4 683 195
- US-A- 4 683 202
- THE EMBO JOURNAL, vol. 5, no. 12, 1986, IRL Press Ltd., Oxford (GB); E. PRINGAULT et al., pp. 3119-3124#
- PROCEEDINGS OF THE NATL. ACADEMY OF SCIENCES USA, vol. 85, no. 14, juillet 1988, Washington, DC (US); W.L. BAZARI et al., pp. 4986-4990#
- THE JOURNAL OF BIOLOGICAL CHEMISTRY, vol. 263, no. 2, 15 janvier 1988, The American Society for Biochemistry and Molecular Biology Inc., Washington, DC (US); E. ANDRE et al., pp. 722-723#
- PROCEEDINGS OF THE NATL. ACADEMY OF SCIENCES USA, vol. 82, décembre 1985, Washington, DC (US); S. ROBINE et al., pp. 8488-8492#
- JOURNAL OF CELL BIOLOGY, (USA), vol. 107, no. 5, novembre 1988, Rockefeller University Press, New York, NY (US); M. ARPIN et al., pp. 1759-1766#
- HUMAN GENETICS, vol. 78, 1988, Springer-Verlag; M.F.ROUSSEAU-MERCK et al., pp. 130-133#
- JOURNAL OF CELL BIOLOGY, vol. 105, no. 4, 1987, New York, NY (US); W.L. BAZARI et al., p. 112A, no. 623#

## Description

L'invention concerne des fragments d'acide nucléique dont la séquence est spécifique du gène de la villine humaine.

Par "séquence spécifique du gène de la villine humaine", on entend, toute séquence contenue dans la séquence codante de ce gène, toute séquence contenue dans l'ARN messager résultant de la transcription de ladite séquence codante, ou encore une séquence complémentaire des précédentes.

L'invention se rapporte également à l'utilisation de ces fragments d'acide nucléique pour détecter l'expression du gène de la villine humaine. Cette application des fragments d'acide nucléique de l'invention permet entre autres de détecter in vitro la présence de cellules tumorales dérivées de tumeurs primaires de la région gastrique ou intestinale ou de métastases, à partir d'un échantillon biologique (tissu ou fluide biologique) prélevé chez un patient au niveau d'organes autres que ceux de la sphère digestive ou intestinale. Une autre application de ces fragments consiste dans la détection du polymorphisme des fragments de restriction du génome humain.

Des travaux réalisés jusqu'à présent ont rapporté l'intérêt de la villine comme marqueur de métastases de tissus autres que ceux de la région digestive ou intestinale. A cet égard la demande de brevet européen n^{o}0206849 déposée le 30 Avril 1986 décrit des moyens pour la détection de la villine à partir d'un prélèvement biologique d'un organe autre que ceux de la sphère digestive ou intestinale. La demande de brevet EP 0206849 tire profit du fait que la villine est de façon générale présente, plus particulièrement dans les bordures en brosse observées à la surface des entérocytes, au niveau du pôle apical des cellules allongées affleurant à la paroi interne de l'intestin et dans les cellules du tubule proximal du rein. La villine n'est en revanche pas détectée dans d'autres organes tels que les poumons, le cerveau etc... Lors de métastases de tumeurs digestives ou intestinales, des cellules produisant de la villine peuvent migrer dans des organes différents. On peut alors détecter la villine dans ces métastases et diagnostiquer une tumeur primaire d'origine digestive ou intestinale.
Ces moyens de diagnostic décrits dans la susdite demande EP 0206849 et destinés à la mise en évidence de la protéine de son messager ou du gène la codant, sont constitués respectivement soit par des anticorps dirigés contre la villine, soit par tout ou partie d'un ADN correspondant à l'ARNm complet de la villine humaine, soit encore par un fragment d'ADNc renfermant au moins une partie de la séquence nucléotidique codant pour les acides aminés de l'extrémité COOH de la villine humaine.

Jusqu'à présent seule cette partie COOH-terminale de l'acide nucléique de la villine avait été séquencée.

La villine appartient de façon générale à la classe des protéines ayant une activité de liaison de l'actine. La villine présente une spécificité qui lui est propre à l'intérieur du groupe des protéines capables de se lier à l'actine. Cette spécificité concerne d'une part sa double fonction in vitro en rapport avec la concentration de calcium du milieu, d'autre part sa distribution tissulaire très spécifique. Néanmoins la villine présente dans certaines régions de sa séquence en acides aminés certaines homologies de séquence avec une autre protéine présente chez les eucaryotes supérieurs, la gelsoline.

En particulier, la comparaison des séquences d'acides aminés de ces protéines montre qu'elles présentent une organisation structurale globale proche, à l'exclusion de la partie COOH terminale, comportant un domaine dupliqué de quatre motifs dont trois sont homologues. Ainsi la villine et la gelsoline présentent, dans leur partie commune, une homologie globale d'environ 5O% en acides aminés, cette homologie étant plus forte au niveau de certaines régions. C'est essentiellement au niveau de la partie COOH-terminale particulière désignée par "head-piece" (région d'extrémité) que la villine se distingue de la gelsoline.

L'invention découle de la découverte après séquençage des régions codant pour la villine que, contre toute attente, la communauté d'organisation de la villine et de la gelsoline et l'homologie de leurs séquences d'acides aminés n'avaient pas d'équivalents au niveau des acides nucléiques codant respectivement pour la villine et la gelsoline.

Les inventeurs ont tiré profit de ces observations pour mettre au point de nouveaux moyens pour détecter de façon spécifique l'expression du gène codant pour la villine humaine. Ces moyens permettent notamment de détecter de façon précoce la présence d'ARN messager (ARNm) résultant de la transcription du gène de la villine humaine.

Les moyens conformes à l'invention pour détecter l'expression du gène codant pour la villine humaine sont constitués par des fragments d'acide nucléique, spécifiques de la séquence codante du gène de la villine humaine ou de la transcription de ce gène.

Il résulte de la constatation faite par les inventeurs qu'un fragment d'acide nucléique comportant au moins 8 nucléotides et issu de l'ADN codant pour la villine humaine ou de l'ARN messager transcrit à partir de cet ADN codant a l'exception de la séquence codant pour la partie "Head piece", peut être spécifique du gène de la villine humaine en particulier distingué de fragments correspondants de l'ADN codant pour la gelsoline.

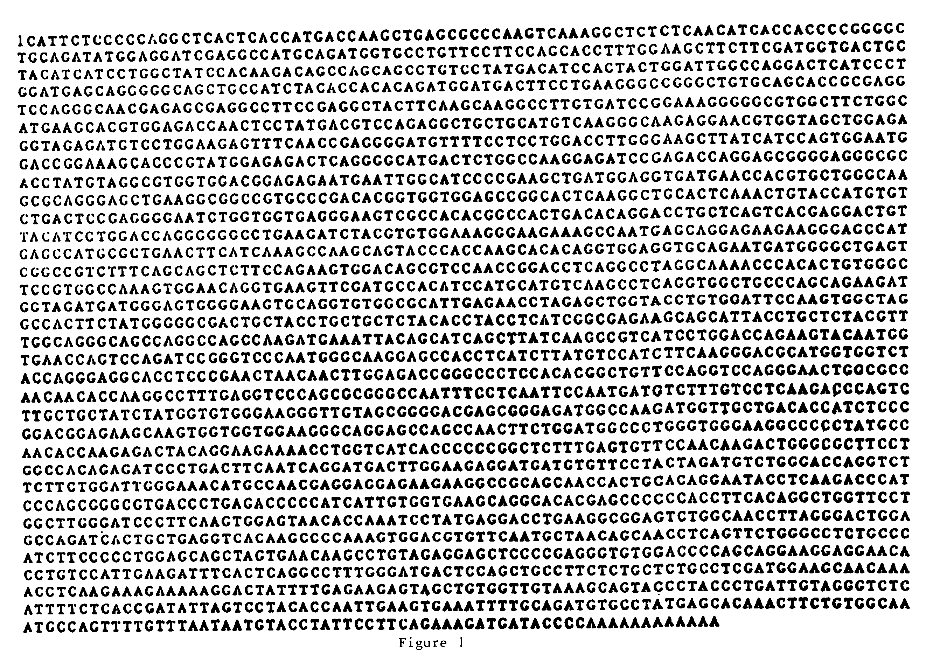

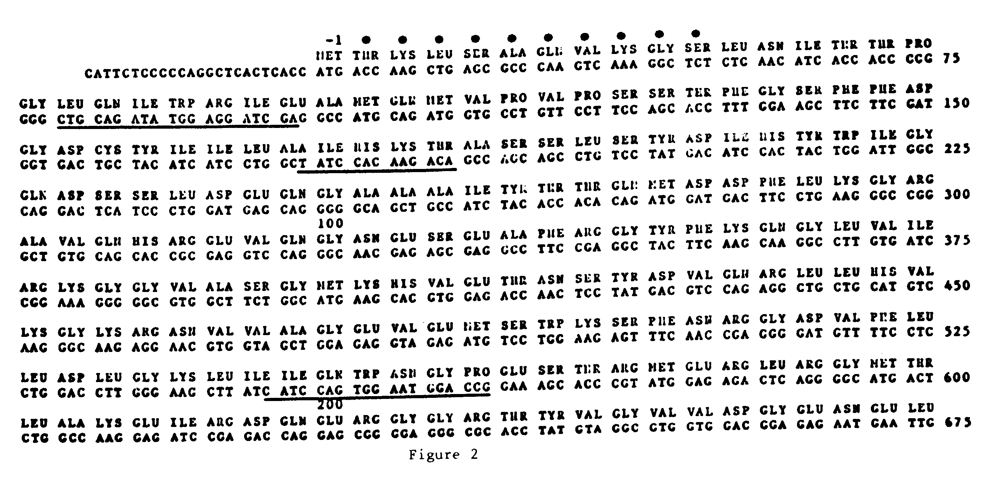

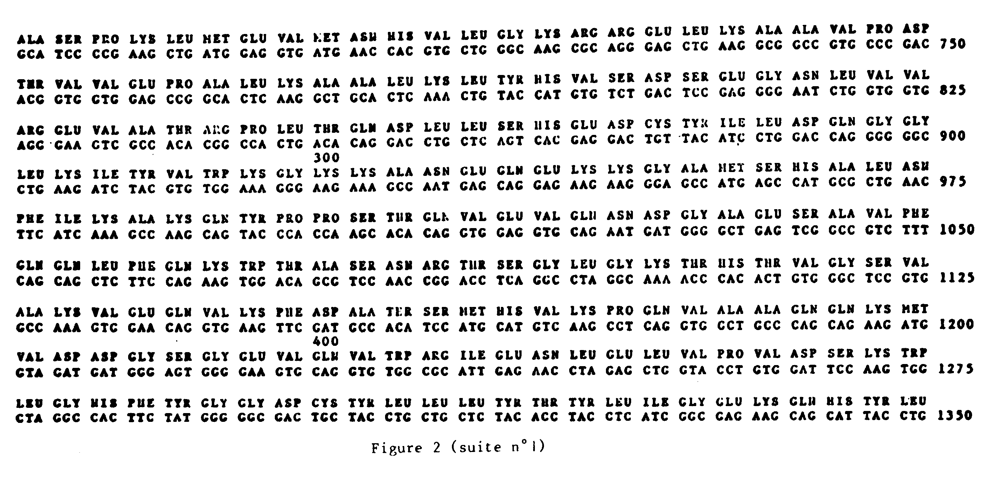

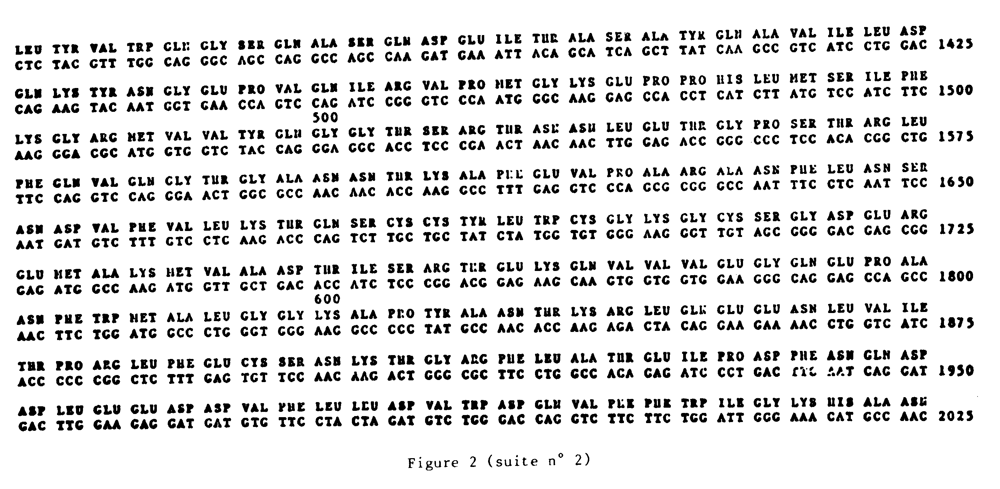

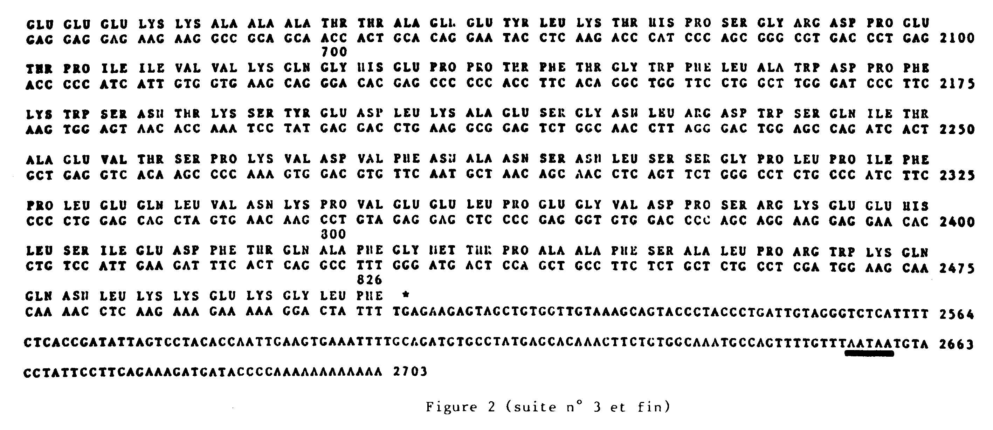

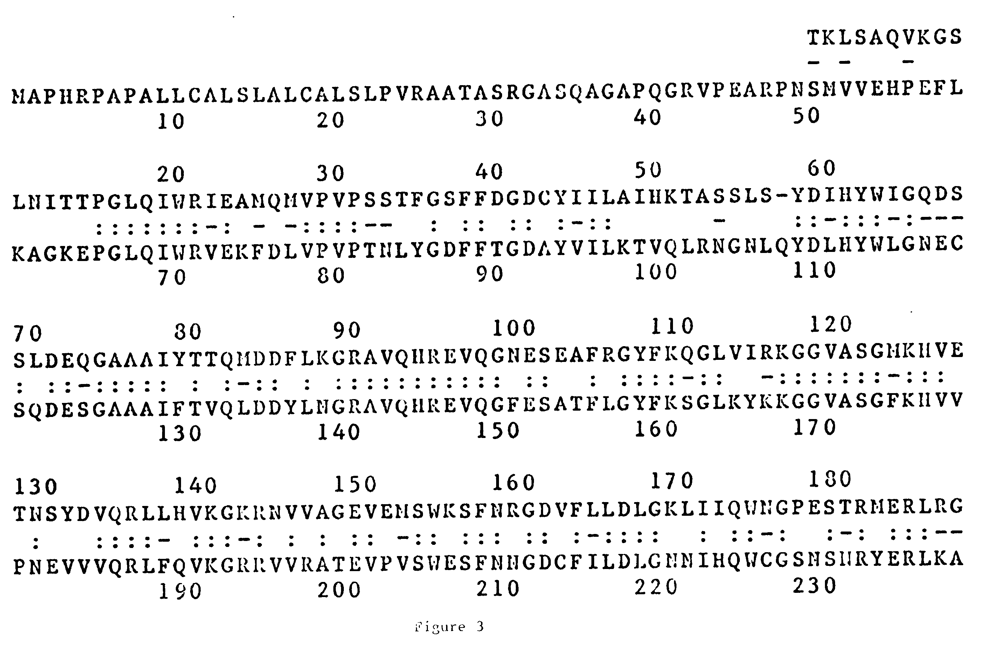

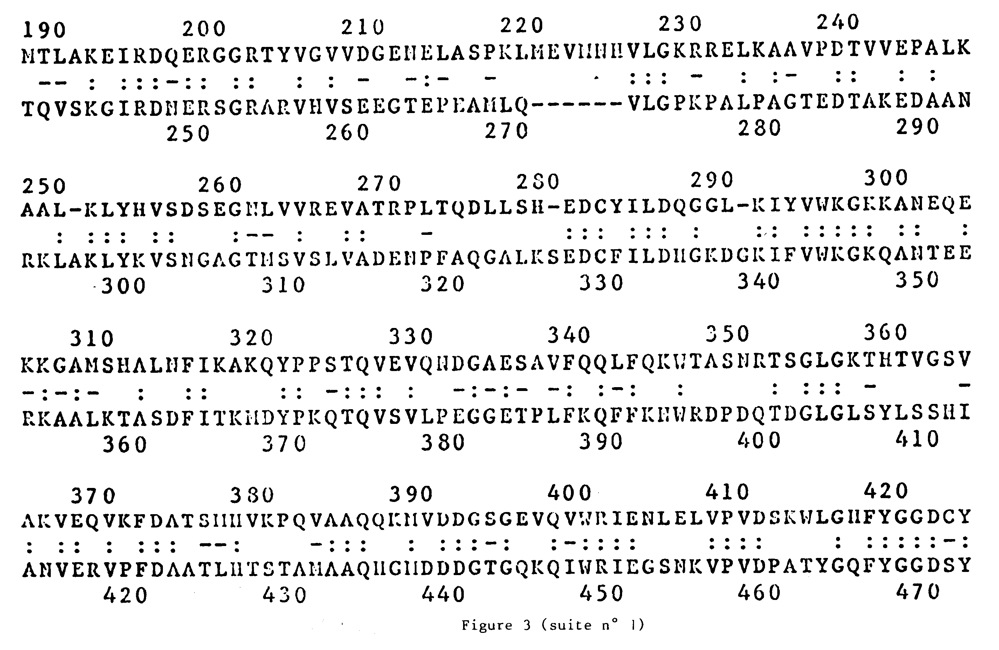

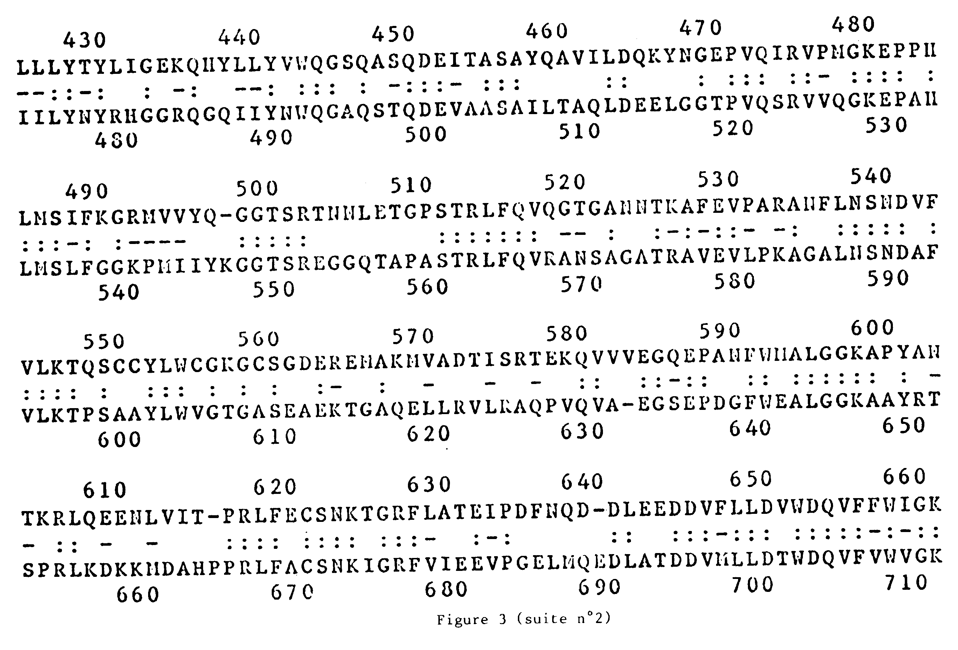

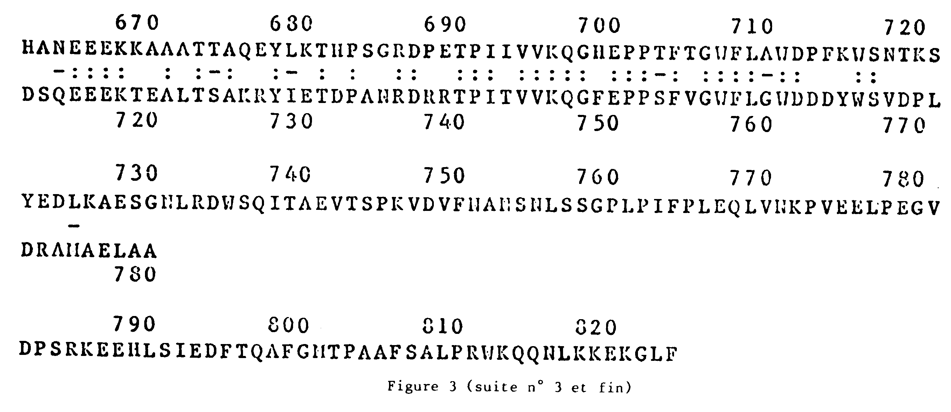

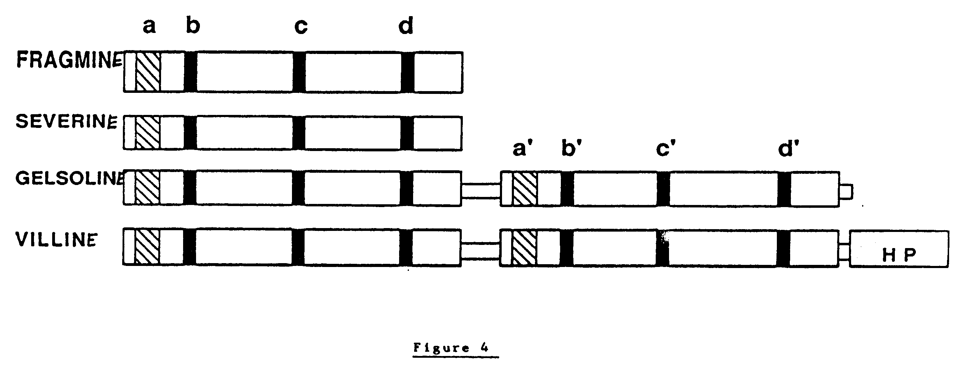

L'invention concerne donc tout fragment d'acide nucléique comprenant un enchaînement de 8 à 40, de préférence 20 à 40 nucléotides, caractérisé en ce que sa séquence est contenue dans la séquence d'ADN codante du gène de la villine humaine représenté à la figure I à l'exception de la séquence codant pour la partie "Head piece". Elle concerne également tout fragment d'ADN exactement complémentaire de l'un des précédents et contenant de ce fait le même nombre de deoxyribonucléotides ainsi que tout fragment d'ARN correspondant et contenant le même nombre de ribonucléotides.

L'invention concerne plus particulièrement les séquences définies ci-dessus sous forme monocaténaire mais également dans le cas des ADN, sous forme bicaténaire.

Les fragments précédemment définis peuvent être isolés par clivage et, le cas échéant émondage par une enzyme appropriée, par exemple Bal 31, à partir du gène codant pour la villine humaine, ou à partir de cDNA préparé à partir de l'ARNm résultant de la transcription de ce gène. Ces fragments peuvent aussi être synthétisés par voie chimique ou par voie enzymatique à partir de la séquence donnée à la figure I qui représente la séquence codante du gène de la villine humaine.

La synthèse par voie chimique d'un des fragments peut être réalisée par mise en oeuvre de la méthode "phosphodiester" dont les étapes sont résumées ci-après :
L'extrémité phosphate-5' de l'un des nucléotides, après protection des groupements fonctionnels, réagit (réaction de condensation) avec l'extrémité hydroxyl-3' d'un autre nucléoside ou nucléotide également protégé en utilisant le dicyclohexylcarbodiimide ou un chlorure d'arylsulphonyle. La chaîne de nucléotides est ainsi allongée par mise en oeuvre d'étapes semblables de condensation.

Des groupements protecteurs des fonctions des nucléotides peuvent par exemple consister en un groupe monomethoxytrityl (MMTr), un groupe acétyl (Ac) ou un groupe anisoyl (An).

La synthèse par voie chimique peut également être réalisée par la méthode "phosphotriester" qui permet de bloquer chaque fonction phosphodiester internucléotide pendant le déroulement de la synthèse de la séquence.

Pour réaliser cette méthode, on utilise un mononucléotide totalement protégé et contenant un groupe phosphotriester-3' masqué.

Une troisième méthode permettant la synthèse par voie chimique d'un fragment d'acide nucléique est la méthode "phosphite-triester". De préférence, on met en oeuvre cette méthode avec l'aide d'un support insoluble sur lequel se fixe l'extrémité 3' de l'oligonucléotide à synthétiser. Cette immobilisation de l'oligonucléotide par couplage permet d'éviter des étapes de purification après chaque cycle de condensation. Pour la réalisation de cette méthode, les mononucléotides appropriés préalablement bloqués sont ajoutés de façon séquentielle et les réactifs, les matériaux de départ non désirés sont éliminés par filtration.

Les groupes protecteurs de l'acide nucléique synthétisé sont éliminés en fin de réaction, l'acide nucléique est ensuite détaché du support et purifié par électrophorèse ou HPLC.

Lorsque l'oligonucléotide synthétisé est fixé sur un support du type polymère dans une colonne, les étapes de filtration peuvent être remplacées par un lavage.

Les méthodes de synthèse chimique décrites peuvent bénéficier de l'enseignement du chapitre 2 "Isolation, Identification and Characterisation of DNA fragments" de l'ouvrage intitulé "From Genes to clones" de E.L. WINNACKER.

Il est entendu que toute région de la séquence codante ou de l'ARNm correspondant peut servir de base à l'obtention d'un fragment d'acide nucléique de l'invention identique ou complémentaire de ces séquences dès lors que la spécificité de l'enchaînement des nucléotides du fragment vis-à-vis de l'ADN codant ou de l'ARNm de la villine est respectée.

L'invention se rapporte aussi à l'application particulière que constitue l'utilisation des fragments d'acides nucléiques définis, pour la constitution d'amorces nucléotidiques (désignées encore par amorces), capables d'induire dans des conditions déterminées, l'initiation de la synthèse de tout ou partie d'un acide nucléique spécifique du gène de la villine.

Par amorce on entend un enchaînement de nucléotides (oligonucléotide) constitué avantageusement par un fragment d'acides nucléiques défini plus haut, notamment tout fragment comportant avantageusement de 8 à 40 nucléotides et de préférence de 20 à 40 nucléotides et répondant aux propriétés suivantes :
- il est capable selon sa nature, d'hybrider spécifiquement avec une séquence correspondante complémentaire soit de l'ADN codant du gène de la villine soit de l'ARNm correspondant à ce gène, soit d'une séquence qui leur est complémentaire,
- il est susceptible d'induire la synthèse d'un acide nucléique complémentaire de celui auquel il s'hybride (ce dernier est alors appelé matrice) au sein d'une solution tamponnée et dans des conditions de pH et de température appropriées pour cette synthèse, en présence d'un agent inducteur de la synthèse, notamment une polymérase ou une réverse transcriptase et en présence des nucléotides appropriés. Le produit de synthèse obtenu à partir du fragment servant d'amorce est appelé produit d'allongement de l'amorce.

Des solutions tamponnées et conditions de pH et de températures appropriées à la production du "produit d'allongement de l'amorce" peuvent être déterminées par référence aux enseignements des brevets suivants : US.4683202 et US.4683195 dont le contenu est incorporé dans la présente demande.

L'amorce se présente sous forme bicaténaire ou monocaténaire. Cette deuxième forme est généralement plus favorable à la qualité requise de l'hybridation avec l'acide nucléique pour que puisse démarrer la synthèse du produit d'allongement à partir de l'amorce et au contact de la matrice.

L'utilisation d'une amorce bicaténaire, dénaturée avant l'étape d'hybridation, peut engendrer une compétition entre, d'une part, la réhybridation de l'amorce avec le brin complémentaire et, d'autre part, l'acide nucléique matrice susceptible de s'apparier l'un des brins de l'amorce.

L'invention concerne plus particulièrement l'application des amorces conformes à l'invention à l'initiation de la synthèse d'une quantité suffisante pour être détectée, de produits d'allongements de l'amorce au contact d'un acide nucléique codant pour la villine humaine, présent dans la séquence de la figure I, ou à partir d'une séquence d'ARNm de transcription du gène de la villine humaine ou à partir des séquences complémentaires des précédentes, présentes dans un échantillon biologique, ces séquences agissant alors comme matrices respectivement complémentaires desdites amorces.

Une telle application permet par exemple la détection d'une altération (mutation, délétion...) sur le fragment d'acide nucléique constituant le produit d'allongement de l'amorce.

En rapport avec ce qui précède, l'invention concerne donc un procédé pour détecter in vitro, la présence soit d'un acide nucléique codant pour la villine humaine soit d'un ARNm de transcription du gène de la villine humaine dans un échantillon biologique suspecté de contenir cet ARNm, comprenant les étapes suivantes :
a/ la mise en contact, en présence de nucléosides triphosphates et d'un agent inducteur de polymérisation, de l'échantillon biologique suspecté de contenir l'ARNm de transcription du gène de la villine humaine, préalablement rendu accessible à une première amorce dans des conditions autorisant l'hybridation entre ces amorces et l'ARNm recherché, et la polymérisation à partir de ces amorces hybridées à l'ARNm, d'acide nucléique complémentaire (ADNc) de l'ARNm, pour produire un duplex formé entre le produit d'allongement de l'amorce, hybridé soit avec le brin d'acide nucléique codant pour la villine humaine, soit avec l'ARNm correspondant lorsqu'ils sont présents dans l'échantillon biologique,
b/ la dénaturation du duplex obtenu à l'étape a/ de façon à "séparer" le produit d'allongement de l'amorce de l'ARNm à détecter,
c/ la mise en contact du produit d'allongement obtenu avec une deuxième amorce (au sens que l'on a donné plus haut de cette expression) ayant une séquence de nucléotides (1) non complémentaire de celle de la première amorce et (2) complémentaire d'une séquence du produit d'allongement précédemment formé,
d/ le cas échéant la répétition des étapes a/ et b/ et c/ en présence des première et deuxième amorces utilisées en excès et des réactifs nécessaires à la production de nouveaux produits d'allongement utilisés à leur tour comme matrice pour des synthèses supplémentaires, jusqu'à obtenir une quantité suffisante pour être détectée de produits d'allongement des amorces utilisées.
e/ la détection de la présence des produits d'allongement caractéristiques de la présence de l'ADN ou de l'ARNm de la villine humaine.

Un tel procédé peut être utilisé par exemple pour la détection in vitro de la présence de cellules tumorales ou de tumeurs, ou encore de métastases de tumeurs, dès lors qu'elles sont caractérisées par la présence anormale de l'ARNm de la villine humaine. A titre d'exemple, on mentionnera les cellules provenant de la tumorigénèse de tissus intestinaux ou rénaux.

La mise en oeuvre du procédé de détection ci-dessus est réalisée à partir d'un échantillon biologique prélevé chez un patient et constitué par exemple par une biopsie, une pièce opératoire, un fluide biologique.

Selon un mode de réalisation particulier du procédé ci-dessus, l'amorce, ou les première et seconde amorces ainsi que les nucléotides sont ajoutés dès la première étape en quantité suffisante pour réaliser plusieurs cycles de synthèse du ou des produits d'allongement, par mise en oeuvre des étapes du procédé ci-dessus défini afin d'obtenir un niveau d'amplification désiré de l'ARNm initialement présent dans l'échantillon biologique.

Selon une variante de réalisation du procédé ces constituants sont incorporés tout au long de la réalisation des étapes du procédé, aussi souvent que nécessaire. Les nucléotides introduits dans l'étape initiale du procédé de détection ci-dessus sont constitués par des déoxynucléotides ou par des ribonucléotides selon que l'on dispose d'amorces constituées respectivement par de l'ADN ou de l'ARN.

L'agent inducteur de la polymérisation à partir de l'amorce hybridée à l'acide nucléique servant de matrice est de façon générale constitué par une ADN-polymérase lorsqu'il s'agit d'induire la synthèse d'ADN, ou par une transcriptase-réverse pour synthétiser de l'ADN à partir d'une matrice d'ARN. A titre d'exemple de polymérases utilisables on mentionnera, l'ADN polymérase de E.coli, le fragment Klenow de cette ADN polymérase, la Taq polymérase ou la transcriptase réverse.Cette énumération n'est naturellement pas exhaustive.

Tout système, toute enzyme pouvant être utilisés comme catalyseur de la polymérisation entrent au contraire dans le cadre de l'invention.

Le duplex obtenu après polymérisation à partir d'une amorce, lorsque le produit d'allongement de l'amorce et la matrice de synthèse sont de nature différente, c'est-à-dire lorsque l'ARNm est initialement présent dans l'échantillon biologique est un hétéroduplex quand le produit d'allongement de l'amorce d'ADN est de l'ADNc, complémentaire de l'ARNm.

Pour plus de détails concernant la technique relative au procédé de détection décrit ci-dessus, ou pour l'élaboration de variantes de ce procédé, l'homme du métier pourra se reporter utilement aux principes décrits dans les brevets US.4.683.202 et US.4.683.195.

La mise en oeuvre du procédé décrit précédemment peut résulter si besoin dans l'amplification d'une partie déterminée de la séquence de l'ARNm de transcription du gène de la villine. Dans ce cas on pourra avoir recours à la mise en oeuvre de deux amorces spécifiques formant les extrémités 5′ et 3′ de la séquence à amplifier.

La taille de la séquence à amplifier est choisie pour permettre la détection de la présence de l'ARNm recherché. La polymérisation à partir d'une amorce peut donc être volontairement limitée par tout moyen approprié.

La détection des produits d'allongement des amorces peut être faite par les techniques classiques de détection. Selon un mode de réalisation de l'invention le procédé de détection de la présence d'ARNm est caractérisé en ce que les nucléotides incorporés pour les besoins de la réalisation du procédé, sont marqués radioactivement et que la détection de la présence de l'ARNm recherché correspond à la détection de la radioactivité des produits d'allongement synthétisés après élimination des nucléotides non incorporés lors de la polymérisation à partir des amorces.

Une autre application des fragments conformes à l'invention concerne leur utilisation pour la détection du polymorphisme génétique humain.

Par détection du polymorphisme génétique humain, on entend à la fois l'identification génétique d'un individu et la détection de certaines pathologies liées à des modifications génétiques concernant le gène de la villine ou un gène lié génétiquement. L'identification d'un individu peut comprendre l'analyse des fragments de restriction contenant tout ou partie du gène de la villine.

Dans le cadre de cette application, les fragments d'acide nucléique définis précédemment sont utilisés à titre de sonde.

Ces sondes sont mises en oeuvre sur un échantillon biologique par exemple un tissu ou un fluide préparé pour être accessible auxdites sondes.

Pour la mise en oeuvre de ces sondes, on se réfèrera à la demande de brevet européen 0186 271.

D'autres caractéristiques et avantages de l'invention apparaîtront dans les exemples qui suivent et dans les figures :
- la figure I représente la séquence codante d'ADN du gène de la villine humaine,
- la figure II comporte la séquence codante de la figure I en correspondance avec la séquence d'acides aminés de la villine humaine,
- la figure III montre la comparaison entre les séquences codantes de la villine et de la gelsoline.
- la figure IV montre les structures comparées de la villine de la gelsoline et de protéines voisines présentes chez les eucaryotes inférieurs.

### SEQUENCAGE DE L'ADNc COMPLEMENTAIRE DE L'ARNm DE LA VILLINE HUMAINE

### Construction d'une banque d'ADNc

L'ARN total a été isolé à partir d'un sous-clone d'une lignée cellulaire HT-29 (HT29-18-C1; Huet et al.,1987) par la méthode à l'isothiocyanate de guanidine (Chirgwin et al.,1979) et enrichi en ARN polyadénylé (ARN poly A⁺) par passage sur une colonne d'oligodT cellulose (Aviv et Leder, 1972).

L'ARN polyadenylé a été fractionné selon la taille sur un gradient de sucrose 5%-20% et les fractions contenant l'ARNm de la villine ont été identifiées par analyse Northern blot en utilisant une sonde ARNc (ARN complémentaire) correspondant à la partie carboxy-terminale de la villine humaine (Pringault et al. 1986, référence 1).

L'ADNc a été synthétisé à partir d'1»g d'ARN polyadénylé conformément à la méthode décrite par Gubler et Hoffmann (1983). Après méthylation de l'ADNc avec une méthylase EcoR1, des linkers EcoR1 ont été ligués aux extrémités franches de l'ADNc, digérés avec EcoR1 et séparés sur une colonne Ultrogel AcA34 (LKB) dans un tampon TE (Tris 20mM, EDTA 1mM). 30ng d'ADNc ont été ligués avec 1»g de l'ADN d'un vecteur λgt10 digéré par EcoR1 et les particules ont été encapsidées in vitro ("in vitro packaging") pour donner une banque d'ADNc contenant 10⁶ recombinants indépendants.

Pour isoler la partie 5′ de l'ADNc de la villine, 3 extensions à partir d'amorces (primer) successives ont été effectuées en utilisant des séquences d'oligonucléotides complémentaires de l'ARNm de la villine. Les sondes d'oligonucléotides sont soulignées dans la figure II. Les oligonucléotides ont été hybridés suivant un rapport molaire de 10:1 avec 2»g d'ARNm polyA⁺ fractionné selon la taille. La synthèse d'ADNc a ensuite été réalisée conformément aux conditions déjà décrites. La banque λgt10 a été criblée avec des sondes ARNc obtenues par transcription in vitro (Melton et al. 1984 référence 2) des clones d'ADNc déjà caractérisés.

### Analyse NORTHERN BLOT

L'ARN poly A⁺ (1,5»g) isolé à partir de la lignée cellulaire HT29-18-C1 a été fractionné par électrophorèse sur gels d'agarose 1% en présence de 1M formaldéhyde (Lehrach et al. 1977 référence 3) et transféré sur nitrocellulose. Les empreintes ont été pré-hybridées à 55°C pendant 16 heures dans 50% de formamide, 4 x SSC, 0.05 M Na₂HPO4 à pH 7,4 dans 1 x de solution Denhardt's, 250 »g/ml d'ADN de sperme de saumon dénaturé et 500»g/ml d'ARNt. L'hybridation avec une sonde ARN marquée au P³² (2x10⁶ c.p.m/ml) a été réalisée pendant 24 heures à 55°C dans la même solution dépourvue d'ARNt. Les empreintes ont été lavées deux fois dans 1xSSC, 0.1% SDS, une fois dans 0.2 x SSC, 0.1% SDS à 65°C et finalement une fois dans 0.1 x SSC, 0.1% SDS pendant 30 min à 70°C.

### Analyse de la séquence.

Des fragments de restriction de l'ADNc ont été sous-clonés dans des dérivés M13mp18-mp19 et séquencés par la méthode didéoxy-de terminaison de chaîne décrite par SANGER et al. (1977 référence 4). Les données sur les séquences des deux brins se recoupent.

### Analyse de la séquence protéique.

La villine humaine a été purifiée par B.West, L.West et M.Mooseker (département de biologie, Université de Yale) à partir de cellules à bords en brosse isolées chez un humain (Carboni et al.1987 référence 5) par des méthodes décrites par Coleman et Mooseker(1985 référence 6). Avant le séquençage 25»g de villine ont été soumis à une électrophorèse sur gel SDS polyacrylamide sur un gel 10% et électrotransféré sur une membrane de fibres de verre (Whatman GF/C) recouverte avec du poly(4-vinyl-N-méthylpyridine). Les détails concernant cette méthode sont donnés dans BAUW et al. (1987 référence 7). La protéine immobilisée a été détectée par un colorant dilué de fluorescamine (1mg/l d'acétone), détachée de la membrane et transférée dans la chambre de réaction d'un séquençeur de protéines en phase gazeuse, Applied Biosystems 470A fonctionnant suivant les instructions du fabricant.

Le séquençage initial conduit à l'obtention d'environ 10% de la protéine déposée sur le gel. On sait que le transfert de protéine est rarement important quantitativement (au plus entre 60 et 90%) et qu'un blocage NH₂-terminal résultant d'un artefact se produit pendant l'électroforèse sur gel (dans la plupart des cas plus de 50% de la protéine est bloqué), voir Moos et al.1988 référence 8). C'est pourquoi la valeur obtenue n'est pas anormalement faible et reflète probablement la séquence NH₂-terminale de la majorité des molécules de villine, plutôt que le résultat d'un clivage protéolytique proche de la partie NH₂ terminale d'une fraction des molécules de villine, qui seraient bloquées par l'extrémité NH₂-terminale.

### REFERENCES BIBLIOGRAPHIQUES:

- (1) Pringault,E. et al 1986. A human villin cDNA clone to investigate the differentiation of intestinal and kidney cells in vivo and in culture. EMBO J.5:3119-3124
- (2) Melton,D.A. et al 1984. Efficient in vitro synthesis of biologically active RNA and RNA hybridization probes from plasmids containing a bacteriophage SP6 promoter. Nucl. Acids Res. 12:7035-7056.
- (3) Lehrach,H. et al 1977. RNA molecular weight determinations by gel electrophoresis under denaturing conditions, a critical reexamination. Biochemistry, 16: 4743-4751.
- (4) Sanger,F.et al. 1977. DNA sequencing with chain-terminating inhibitors. Proc.Nat.Acad.Sci.74:5463-5467.
- (5) Carboni,J.M.et alm.1987. Characterization of intestinal brush border cytoskeletal proteins of normal and neoplastic human epithelial cells : a comparison with the avian brush border. Am.J.Pathol. 129 : 589-600.
- (6) Coleman,T.R. et al. 1985. Effects of actin filament cross-linking and filament length on actin-myosin interaction.J. Cell Biol.101 : 1850-1857.
- (7) Bauw,G.et al. 1987. Alterations in the phenotype of plan cells studied by NH₂-terminal amino acid-sequence analysis of proteins electroblotted from two-dimensional gel-separated total extracts. Proc.Natl.Acad.Sci.84 : 4806-4810.
- (8) Moos,M.Jr.et al. 1988. Reproducible high yield sequencing of proteins electrphoretically separated and transferred to an inert support. J.Biol.Chem.263:6005-6008.

## Revendications

1. Fragment d'acide nucléique caractérisé en ce qu'il comprend de 8 à 40 nucléotides et en ce que sa séquence est contenue soit dans la séquence d'ADN codante, à l'exception de la séquence codant pour la partie "Head piece", du gène de la villine humaine soit dans un fragment d'ADN exactement complémentaire de l'un des précédents et contenant de ce fait le même nombre de deoxynucléotides, soit dans tout fragment d'ARN correspondant et contenant le même nombre de ribonucléotides.

2. Fragment d'acide nucléique selon la revendication 1, caractérisé en ce que sa séquence comprend de 20 à 40 nucléotides.

3. Fragment d'acide nucléique, caractérisé en ce qu'il comprend au moins 8 nucléotides et en ce que sa séquence est contenue soit dans la séquence d'ADN codante, à l'exception de la séquence codant pour la partie "Head piece", du gène de la villine humaine soit dans un fragment d'ADN exactement complémentaire de l'un des précédents et contenant de ce fait le même nombre de deoxynucléotides, soit dans tout fragment d'ARN correspondant et contenant le même nombre de ribonucléotides, ce fragment étant en outre distinct par rapport à un fragment correspondant de l'ADN codant pour la gelsoline.

4. Application des fragments d'acides nucléiques selon l'une quelconque des revendications 1 ou 2 en tant qu'amorce pour l'initiation de la synthèse d'une quantité suffisante pour être détectée, de produits d'allongement de l'amorce au contact d'un acide nucléique codant pour la villine humaine présent dans la séquence codante présentée à la figure I, ou à partir d'une séquence d'ARNm de transcription du gène de la villine humaine ou à partir des séquences complémentaires des précédentes, présentes dans un échantillon biologique, ces séquences agissant alors comme matrices respectivement complémentaires desdites amorces.

5. Procédé pour détecter in vitro, la présence soit d'un acide nucléique codant pour la villine humaine soit d'un brin d'ARNm de transcription du gène de la villine humaine dans un échantillon biologique, suspecté de contenir cet ARNm, comprenant les étapes suivantes :
a/ la mise en contact, en présence de nucléosides triphosphates et d'un agent inducteur de polymérisation, de l'échantillon biologique suspecté de contenir l'ARNm de transcription du gène de la villine humaine, préalablement rendu accessible à une première amorce constituée par un fragment d'acide nucléique selon l'une quelconque des revendications 1 ou 2, dans des conditions autorisant l'hybridation entre cette amorce et l'ARNm recherché, et la polymérisation à partir de cette amorce hybridée à l'ARNm, d'acide nucléique complémentaire (ADNc) de l'ARNm, pour produire un duplex formé entre le produit d'allongement de cette première amorce, hybridé soit avec le brin d'acide nucléique codant pour la villine humaine soit avec l'ARNm correspondant lorsqu'ils sont présents dans l'échantillon biologique,
b/ la dénaturation du duplex obtenu à l'étape a/ de façon à "séparer" le produit d'allongement des amorces, de l'ARNm à détecter,
c/ la mise en contact du produit d'allongement obtenu avec une deuxième amorce (au sens que l'on a donné plus haut de cette expression) ayant une séquence de nucléotides (1) non complémentaire de celle de la première amorce et (2) complémentaire d'une séquence du produit d'allongement précédemment formé,
d/ le cas échéant la répétition des étapes a/, b/ et c/ en présence des première et deuxième amorces utilisées en excès et des réactifs nécessaires à la production de nouveaux produits d'allongement utilisés à leur tour comme matrice pour des synthèses supplémentaires, jusqu'à obtenir une quantité suffisante pour être détectée, de produits d'allongement des amorces utilisées,
e/ la détection de la présence des produits d'allongement caractéristiques de la présence de l'ADN ou de l'ARNm de la villine humaine.

6. Procédé selon la revendication 5, caractérisé en ce que les nucléosides triphosphates sont marqués radioactivement et que la détection de la présence de l'ARNm recherché correspond à la détection de la radioactivité des produits d'allongement synthétisés, après élimination des nucléosides triphosphates non incorporés lors de la polymérisation à partir des amorces.

7. Procédé selon la revendication 5, caractérisé en ce que les nucléosides triphosphates sont des déoxyribonucléosides triphosphates et en ce que l'agent inducteur est une DNA polymérase ou une transcriptase réverse.

8. Procédé selon l'une quelconque des revendications 5 ou 6, pour la détection d'une tumeur ou d'une métastase, caractérisé en ce qu'il est réalisé sur une biopsie dont les ARNm de transcription du gène de la villine éventuellement présents ont été rendus accessibles aux amorces utilisées.

9. Sonde pour la détection du polymorphisme des fragments de restriction du génome humain caractérisée en ce qu'elle comprend un fragment d'acide nucléique selon l'une quelconque des revendications 1 ou 2.

## Claims

1. Nucleic acid fragment characterized in that it comprises from 8 to 40 nucleotides and in that its sequence is contained either in the DNA sequence coding for the gene for human villin, with the exception of the sequence coding for the "head piece", or in an DNA fragment exactly complementary to one of the foregoing and consequently containing the same number of deoxynucleotides, or in any corresponding RNA fragment containing the same number of ribonucleotides.

2. Nucleic acid fragment according to Claim 1, characterized in that its sequence comprises from 20 to 40 nucleotides.

3. Use of the nucleic acid fragments according to any one of the Claims 1 or 2 as primers for the initiation of the synthesis of a detectable quantity of elongation products of the primer in contact with a nucleic add coding for human villin present in the coding sequence presented in Figure I, or from a mRNA sequence resulting from the transcription of the gene for human villin or from sequences complementary to the foregoing, these sequences then acting as complementary matrices of the said primers.

4. Procedure for in vitro detection of the presence of either a nucleic acid coding for human villin or an mRNA strand resulting from the transcription of the gene for human villin in a biological sample suspected to contain this mRNA, comprising the following steps :
a/ the placing of a biological sample suspected to contain the mRNA resulting from the transcription of the gene for human villin, after it has been made accessible, in contact with a first primer constituted by a nucleic add fragment according to either of the Claims 1 or 2, in the presence of nucleoside triphosphates and polymerization-inducing agent and under conditions permitting the hybridization between this primer and the mRNA under investigation, and the polymerization from this primer hybridized to the mRNA, the nucleic add complementary (cDNA) to this mRNA, in order to produce a duplex formed between the elongation product of this first primer, hybridized either with the nucleic acid strand coding for human villin or with the corresponding mRNA when they are present in the biological sample,
b/ denaturation of the duplex obtained in step a/ so as to "separate" the elongation product of the primers from the mRNA to be detected,
c/ the placing of the elongation product obtained in contact with a second primer (in the sense given above to this expression) having a nucleotide sequence (1) not complementary to that of the first primer and (2) complementary to a sequence of the previously formed elongation product,
d/ optionally, the repetition of the steps a/, b/ and c/ in the presence of the first and second primers used in excess and the reagents necessary for the production of more elongation products used in turn as matrix for additional syntheses until a detectable quantity of the elongation products of the primers used is obtained,
e/ detection of the presence of the elongation products characteristic of the presence of the DNA or mRNA of human villin.

5. Procedure according to Claim 4, characterized in that the nucleoside triphosphates are radioactively labelled and that the detection of the presence of the mRNA under investigation corresponds to the detection of the radioactivity of the elongation products synthesized, after removal of the nucleoside triphosphates not incorporated during polymerization from the primers.

6. Procedure according to Claim 4, characterized in that the nucleoside triphosphates are deoxyribonucleoside triphosphates and in that the polymerization-inducing agent is a DNA polymerase or a reverse transcriptase.

7. Procedure according to either of the Claims 4 or 5 for the detection of a tumor or metastasis, characterized in that it is performed on a biopsy whose mRNAs resulting from the transcription of the villin gene possibly present have been made accessible to the primers used.

8. Probe for the detection of polymorphism of the restriction fragments of the human genome characterized in that it comprises a nucleic acid fragment according to either of the Claims 1 or 2.

9. Nucleic acid fragment, characterized in that it comprises at least 8 nucleotides and in that its sequence is contained either in the DNA sequence coding for the gene for human villin, with the exception of the sequence coding for the "head piece", or in a DNA fragment exactly complementary to one of those previously mentioned and which consequently contains the same number of nucleotides, or in any corresponding RNA fragment containing the same number of ribonucleotides, this fragment being, in addition, distinct from a fragment corresponding to the DNA coding for gelsoline.

## Patentansprüche

1. Nukleinsäurefragment, dadurch gekennzeichnet, daß es 8 bis 40 Nukleotide umfaßt, und daß seine Sequenz entweder in der kodierenden DNS-Sequenz enthalten ist, mit Ausnahme der kodierenden Sequenz für das "Head piece" Teil des menschlichen Villingens, oder in einem DNS Fragment, welches zu einem der vorausgegangenen genau komplementär ist und daher die gleiche Zahl Deoxynukleotide enthält, oder in jedem entsprechenden RNS-Fragment, das die gleiche Zahl Ribonukleotiden enthält.

2. Nukleinsäurefragment gemäß Anspruch 1, dadurch gekennzeichnet, daß seine Sequenz 20 bis 40 Nukleotide umfaßt.

3. Anwendung der Nukleinsäurefragmente gemäß irgendeinem der Ansprüche 1 oder 2 als Auslöser für das Einleiten der Synthese einer für den Nachweis ausreichenden Menge von Verlängerungsprodukten des Auslösers im Kontakt mit einer für das vorhandene menschliche Villin kodierenden Nukleinsäure, die in der in Figur I dargestellten Kodiersequenz vorhanden ist, oder ausgehend von einer RNSm für die Transkription des menschlichen Villingens oder von den Komplementärsequenzen der vorherigen Sequenzen, die in einem biologischen Muster vorhanden sind, wobei diese Sequenzen dann jeweils als Komplementärmatrizen der besagten Auslöser wirken.

4. Verfahren zum in vitro Nachweis entweder des Vorhandenseins einer für das menschliche Villin kodierenden Nukleinsäure oder eines RNSm-Stranges für die Transkription des menschlichen Villingens in einem biologischen Muster, von dem vermutet wird, das es diese RNSm enthält, mit den folgenden Schritten:
a/ das Zusammenbringen, in Gegenwart von Triphosphatnukleosiden, des biologischen Musters von dem vermutet wird, das es die RNSm für die Transkription des menschlichen Villingens enthält, das vorher durch ein Nukleinsäurefragment gemäß irgendeinem der Ansprüche 1 oder 2 einem ersten Auslöser zugänglich gemacht wurde unter Bedingungen, welche die Hybridisierung zwischen diesem Auslöser und der gesuchten RNSm und die Polymerisierung von zur RNSm komplementären Nukleinsäure (DNSc), ausgehend von diesem hybridisierten Auslöser der RNSm, ermöglichen, um ein Duplex herzustellen, das aus dem Verlängerungsprodukt dieses ersten Auslösers, welches entweder mit dem für das menschliche Villin kodierenden Nukleinsäuretrang oder mit der entsprechenden RNSm hybridisiert wurde, wenn sie im biologischen Muster vorhanden sind,
b/ die Denaturierung des im Schritt a/ hergestellten Duplex, um das Verlängerungsprodukt der Auslöser und die nachzuweisende RNSm zu "trennen",
c/ das Zusammenbringen des Verlängerungsproduktes, das mit einem zweiten Auslöser (in dem Sinne, der diesem Ausdruck weiter oben gegeben wurde) erhalten wurde, welcher eine Nukleotidensequenz (1) hat, die derjenigen der ersten Auslöser nicht komplementär ist und (2) komplementär zu einer Sequenz des vorher gebildeten Verlängerungsproduktes ist,
d/ gegebenenfalls, die Wiederholung der Schritte a/, b/ und c/ in Gegenwart der ersten und zweiten, überschüssig angewandten Auslöser und der Reagenzien, die zur Herstellung neuer Verlängerungsprodukte erforderlich sind, welche selbst als Matrix für zusätzliche Synthesen verwendet werden, bis eine zum Nachweis ausreichende Menge von Verlängerungsprodukten der angewandten Auslöser erhalten wird,
e/ der Nachweis der für das Vorhandensein der DNS oder der RNSm des menschlichen Villins charakteristischen Verlängerungsprodukte.

5. Verfahren gemäß Anspruch 4, dadurch gekennzeichnet, daß die Triphosphatnukleosiden radioaktiv markiert werden, und daß der Nachweis für das Vorhandensein der gesuchten RNSm dem Nachweis der Radioaktivität der synthetisierten Verlängerungsprodukte entspricht, nach Entfernung der bei der Polymerisierung aus den Auslösern nicht aufgenommenen Triphosphatnukleosiden.

6. Verfahren gemäß Anspruch 4, dadurch gekennzeichnet, daß die Triphosphatnukleosiden Triphosphatdeoxyribonukleosiden sind, und daß es sich beim induzierenden Agent um eine DNS Polymerase oder um eine inverse Transkriptase handelt.

7. Verfahren gemäß irgendeinem der Verfahren 4 oder 5, für den Nachweis eines Tumors oder einer Metastase, dadurch gekennzeichnet, daß er an einer Biopsie durchgeführt wird, deren eventuell vorhandene Transkriptions-RNSm des Villingens den angewandten Auslösern zugänglich gemacht wurden.

8. Sonde für den Nachweis des Polymorphismus der Restriktionsfragmente des menschlichen Genoms, dadurch gekennzeichnet, daß sie ein Fragment Nukleinsäure gemäß irgendeinem der Ansprüche 1 oder 2 enthält.

9. Nukleinsäurefragment, dadurch gekennzeichnet, daß es mindestens 8 Nukleotiden umfaßt, und daß seine Sequenz entweder in der kodierenden DNS-Sequenz enthalten ist, mit Ausnahme der kodierenden Sequenz für das "Head piece" Teil des menschlichen Villingens, oder in einem DNS Fragment, welches zu einem der vorausgegangenen genau komplementär ist und daher die gleiche Zahl Deoxynukleotide enthält, oder in jedem entsprechenden RNS-Fragment, das die gleiche Zahl Ribonukleotiden enthält, wobei sich dieses Fragment außerdem von einem Fragment unterscheidet, welches der das Gelsolin kodierenden DNS entspricht.
